(19)

Europäisches
Patentamt
European
Patent Office
Office européen
des brevets

(11) **EP 4 475 134 A1**

(12) **EUROPEAN PATENT APPLICATION**

(43) Date of publication:
**11.12.2024 Bulletin 2024/50**

(21) Application number: **23195897.6**

(22) Date of filing: **07.09.2023**

(51) International Patent Classification (IPC):
**G16H 15/00** (2018.01)   **G16H 30/40** (2018.01)
**G16H 50/20** (2018.01)

(52) Cooperative Patent Classification (CPC):
**G16H 50/20; G16H 30/40**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **07.06.2023 PCT/CN2023/202309**

(71) Applicant: **Koninklijke Philips N.V.
5656 AG Eindhoven (NL)**

(72) Inventors:
• **FU, Yong
  Eindhoven (NL)**

• **TIAN, Cong
  Eindhoven (NL)**
• **JIANG, Zeyu
  5656AG Eindhoven (NL)**
• **YUAN, yana
  Eindhoven (NL)**
• **LUO, Zhongchi
  Eindhoven (NL)**

(74) Representative: **Philips Intellectual Property &
Standards
High Tech Campus 52
5656 AG Eindhoven (NL)**

(54) **PREDICTING THE PRESENCE OF ACUTE RESPIRATORY DISTRESS SYNDROME**

(57)     A mechanism for predicting acute respiratory distress syndrome (ARDS) in an individual. Individual data of the individual is processed, using different machine-learning methods, to produce a plurality of feature vectors. The feature vectors are fused using a fusion algorithm to produce a fused feature vector. The fused feature vector is processed using another machine-learning method to produce a predictive indicator of ARDS. The fusion algorithm is trained using at least one loss function that is independent of the performance of the machine-learning algorithm that produces the predictive indicator from the fused feature vector.

FIG. 4

**Description**

FIELD OF THE INVENTION

The present invention relates to the field of generating predictive indicators.

BACKGROUND OF THE INVENTION

Acute respiratory distress syndrome (ARDS) is a severe form of acute lung injury (ALI), which occurs when fluid builds up in the lungs of an individual and deprives the organs of the oxygen they need to function. There is therefore a need to generate accurate predictive indicators of the occurrence of ARDS in order to prompt or alert a clinician to check for the presence of ARDS.

One of the most common approaches for manually checking for the presence of ARDS is to make use of The Berlin Definition, which defines a number of parameters that characterize or indicate the occurrence of ARDS.

However, existing automated approaches typically only make use of non-imaging or non-image data, such as vital sign or lab test values. However, it is recognized that medical image data (e.g., chest X-ray data) can play an important role in accurate identification of potential ARDS, as such image data may provide clues as to the etiology and chronicity of the condition. Medical image data may also have a role in the monitoring of ARDS and in identifying potential clinical complications.

There is therefore a clear desire for a procedure for producing a predictive indicator of ARDS that takes account of both medical image data and non-image medical data. More particularly, it would be desirable to provide an accurate and power-efficient approach for producing such a predictive indicator of ARDS.

SUMMARY OF THE INVENTION

The invention is defined by the claims.

According to examples in accordance with an aspect of the invention, there is provided a computer-implemented method of generating a predictive indicator of acute respiratory distress syndrome for an individual.

The computer-implemented method comprises: obtaining individual data of the individual comprising: medical image data of the individual; and non-image medical data of the individual; processing the medical image data using a first machine-learning method to produce a first feature vector; processing the non-image data of the individual using a second machine-learning method to produce a second feature vector; fusing the first feature vector and the second feature vector, using a fusion algorithm, to form a fused feature vector representing the information of both the first feature vector and the second feature vector; and processing the fused feature vector using a third machine-learning algorithm to generate a predictive indicator that indicates whether or not the individual has acute respiratory distress syndrome.

The fusion algorithm is a trainable algorithm and is trained using at least one measure of loss produced by at least one loss function, wherein each loss function processes a plurality of training fused feature vectors, independently of the third machine-learning algorithm, to produce a respective measure of loss.

The predictive indicator may take the form of binary, categorical or numerical data. For instance, the predictive indicator may be a binary predictor of whether or not the individual has acute respiratory distress syndrome. As another example, the predictive indicator may categorize the likelihood that individual has acute respiratory distress syndrome (e.g., "Not Likely", "Less Likely than Unlikely", "As Likely as Not Likely", "More Likely than Unlikely, "Likely" etc.). In yet another example, the predictive indicator is a predicted probability that the individual has acute respiratory distress syndrome (e.g., on a scale of 0-1, 0-10, 0-100, 1-10, 1-100 and so on).

In the context of the present disclosure, medical data is any data that is relevant for making a medical diagnosis or assessment of the individual. This may include demographic data (e.g., age, gender and so on), patient history information (e.g., length of stay, past medical treatments and/or diagnoses and so on) and/or physiological information (e.g., vital sign information such as a heart rate, respiratory rate and so on).

The fusion algorithm may comprise an autoencoder neural network.

In some examples, each training fused feature vector is associated with a respective individual and a respective predictive indicator that indicates whether or not the respective individual has acute respiratory distress syndrome.

In some examples, the at least one loss function comprises a first loss function that: determines, for each training fused feature vector in a first subset of the plurality of training fused feature vectors, a first measure of difference between said training fused feature vector and another training fused feature vector associated with a same or similar predictive indicator to the predictive indicator of said fused feature vector; and sums or averages the determined first measures of difference to produce a first measure of loss.

The fusion algorithm may be trained to target decreasing the first measure of loss.

The at least one loss function may comprise a second loss function that: determines, for each training fused feature vector in a second subset of the plurality of training fused feature vectors, a second measure of difference between said training fused feature vector and another training fused feature vector associated with a different predictive indicator to the predictive indicator of said fused feature vector; and sums or averages the determined second measures of difference to produce a second measure of loss.

In some examples, the fusion algorithm is trained to target increasing the second measure of loss.

In some examples, the plurality of training fused feature vectors is produced by, for each of a first plurality of instances of training individual data comprising training medical image data and training non-image medical data: processing the training medical image data using the first machine-learning method to produce a training first feature vector; processing the training non-image medical data using the second machine-learning method to produce a training second feature vector; and fusing the training first feature vector and the training second feature vector using the fusion algorithm to produce the training fused feature vector.

The at least one loss function may comprise a third loss function that: for each training fused feature vector in a third subset of the plurality of training fused feature vectors: processing the training fused feature vector using a reconstruction algorithm to produce a reconstructed first feature vector and a reconstructed second feature vector; determining a third measure of difference between the reconstructed first feature vector and the training first feature vector that was fused to produce the training fused feature vector; and determining a fourth measure of difference between the reconstructed second feature vector and the training second feature vector that was fused to produce the training fused feature vector, and summing and/or averaging each third measure of difference and/or fourth measure of difference to produce a third measure of loss.

The fusion algorithm may be trained to target decreasing the third measure of loss.

In some embodiments, the fusion algorithm is trained using at least one further measure of loss produced by a further loss function; the further loss function is configured to process a plurality of instances of training data, each instance of training data comprising: training individual data comprising training medical image data and training non-image medical data of a training subject; and a training predictive indicator that indicates whether or not the training individual has acute respiratory distress syndrome, the further loss function comprises: for each instance of training data: processing the training medical image data and the training non-image medical data, using the first machine-learning method, the second machine-learning method, the fusion algorithm and the third machine-learning method, to produce an intermediate predictive indicator; determining a fifth measure of difference between the intermediate predictive indicator and the training predictive indicator; and summing and/or average each fifth measure of difference to produce a further measure of loss.

The method may further comprise processing the non-image data of the individual using a fourth machine-learning method to produce a third feature vector, wherein fusing the first feature vector and the second feature vector comprises fusing the first feature vector, the second feature vector and the third feature vector, using a fusion algorithm, to form a fused feature vector to represent the information of the first feature vector, the second feature vector and the third feature vector; the second feature vector represents static information of the individual, being information that does not substantially change over the course of a medical treatment; and the third feature vector represents dynamic information of the individual, being information that is expected to change throughout the course of a medical treatment.

The first machine-learning algorithm, the second machine-learning algorithm and/or the third machine-learning algorithm may be trained using the at least one measure of loss produced by the at least one loss function.

There is also proposed a computer-implemented method for training a fusion algorithm for use in a previously described method. The computer-implemented method comprises training the fusion algorithm using at least one measure of loss produced by at least one loss function that processes a plurality of training fused feature vectors independently of the third machine-learning algorithm.

There is also proposed a computer program product comprising computer program code means which, when executed on a computing device having a processing system, cause the processing system to perform all of the steps of any herein disclosed method.

There is also provided a processing system for generating a predictive indicator of acute respiratory distress syndrome for an individual.

The processing system is configured to: obtain individual data of the individual comprising: medical image data of the individual; and non-image medical data of the individual; process the medical image data using a first machine-learning method to produce a first feature vector; process the non-image data of the individual using a second machine-learning method to produce a second feature vector; fuse the first feature vector and the second feature vector, using a fusion algorithm, to form a fused feature vector to represent the information of the first feature vector and the second feature vector; and process the fused feature vector using a third machine-learning algorithm to generate a predictive indicator that indicates whether or not the individual has acute respiratory distress syndrome.

The fusion algorithm is a trainable algorithm and is trained using at least one measure of loss produced by at least one loss function, wherein each loss function processes a plurality of training fused feature vectors, independently of the third machine-learning algorithm, to produce a respective measure of loss.

The processing system may be appropriately adapted to carry out the function(s) of any herein disclosed method, and vice versa.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates a proposed workflow;
Fig. 2 illustrates a fusion algorithm;
Fig. 3 illustrates a fusion algorithm and a reconstruction algorithm; and
Fig. 4 is a flowchart illustrating a proposed method.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for predicting acute respiratory distress syndrome (ARDS) in an individual. Individual data of the individual is processed, using different machine-learning methods, to produce a plurality of feature vectors. The feature vectors are fused using a fusion algorithm to produce a fused feature vector. The fused feature vector is processed using another machine-learning method to produce a predictive indicator of ARDS. The fusion algorithm is trained using at least one loss function that is independent of the performance of the machine-learning algorithm that produces the predictive indicator from the fused feature vector.

Embodiments are based on the realization that improving the performance of the fusion algorithm, e.g., so that the fused feature vector more accurately contains the information of the individual data, will improve the accuracy of predicting ARDS.

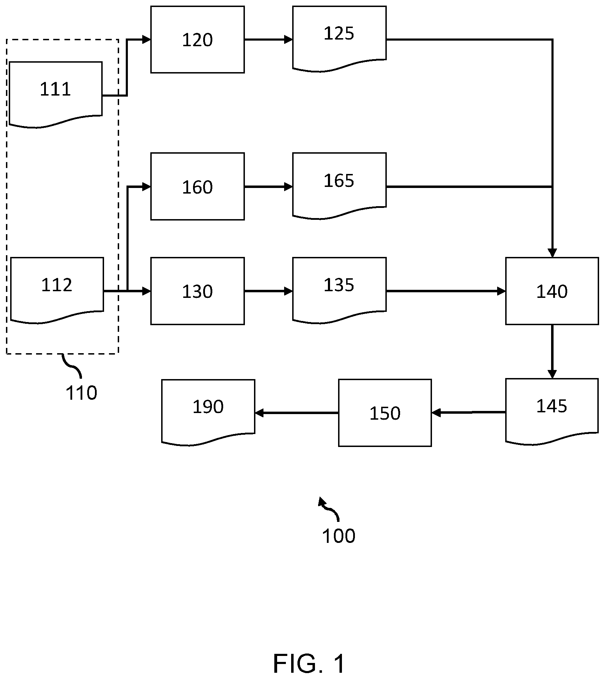

Fig. 1 conceptually illustrates a workflow 100 for generating a predictive indicator 190 of acute respiratory distress syndrome (ARDS) for an individual. The workflow processes individual data 110 of the individual to produce the predictive indicator 190.

Individual data 110 of the individual comprises medical image data 111 and non-medical image data 112.

The medical image data may, for instance, comprise medical image data containing a representation of at least a thorax of the individual. The medical image data may, for instance, comprise ultrasound image data, X-ray image data, CT image data, MR image data, PET image data and so on. Any modality or form of medical image data can be used in proposed concepts.

Embodiments are particularly advantageous when the medical image data comprises X-ray image data of the thorax (or chest) or the individual. This is because X-ray image data has been shown to contain reliable and consistent representations or indicators of ARDS. However, other forms of image data (e.g., of the thorax or other respiratory elements) also contain indicators of ARDS.

The non-image medical data 112 may, for instance, comprise static information and/or dynamic information. Static information includes any data or information that is expected to not undergo substantial change during a clinical stay (e.g., demographic information or the like). Dynamic information includes any data or information that is expected to change or vary through a clinical stay, such as values of physiological parameters (e.g., vital sign data), results of one or more lab tests and so on. An alternative label for the dynamic information is time-series information, e.g., as it is information or data that can be associated with one or more time stamps.

Some examples of static information include: age, gender, ethnicity, past medical history, length of stay. Some examples of dynamic information include measurements of one or more physiological parameters of the individual, such as those derived from laboratory tests (e.g., glucose, P/F ratio) and those derived from direct individual monitoring (e.g., heart rate, respiration rate, oxygen saturation).

The medical image data for the individual may, for instance, be stored in a picture archiving and communication system (PACS) or may be obtained directly from the medical imaging device. The non-medical image data may, for instance, be stored in one or more medical records of the individual, e.g., stored in a clinical information system, from which it can be obtained.

The workflow 100 comprises processing the medical image data using a first machine-learning method 120 to produce a first feature vector 125. The workflow 100 also comprises processing the non-image medical data using a second machine-learning algorithm 130 to produce a second feature vector 135. The second machine-learning method is separate and/or different to the first machine-learning method.

The image medical data and/or non-image medical data may be pre-processed before being input to their respective machine-learning methods.

For instance, a histogram equalization and/or Gaussian blur may be used to pre-process the medical data rays to yield an expanded range of intensity and modestly increased contrast for the first machine-learning method.

As another example, the non-image medical data may be preprocessed by performing one or more processes for: handling missing values; averaging values; removing outliers; and/or feature encoding/normalization. Before the multimodal model training phrase, it is possible to link each unique ICU stay to radiographic studies.

The first and second machine-learning methods may be any suitable machine-learning method that is able to produce a feature vector (e.g., a reduced feature vector). By way of example, a convolutional neural network (CNN) may be used as the first machine-learning algorithm to extract significant features from the image data. The second machine-learning algorithm may, for instance, comprise a neural network comprising fully connected layers or a long short-term memory (LSTM) network.

The workflow also comprises fusing the first feature vector and the second feature vector using a fusion algorithm 140. The fusion algorithm forms a fused feature vector 145 that represents the information of or contained in both the first feature vector 125 and the second feature vector. The fused feature vector 145 may, for instance, be smaller than the combination of the first 125 and second 135 feature vector.

The fusion algorithm 140 may, for instance, be a trainable algorithm. Thus, the fusion algorithm may itself be a machine-learning method. As an example, the fusion algorithm may be or comprise an autoencoder neural network.

The workflow then processes the fused feature vector 145 using a third machine-learning method 150 to generate the predictive indicator 190 that indicates whether or not the individual has acute respiratory distress syndrome. The predictive indicator may be in the form of a classification (e.g., a binary or a categorical classification) or a probability. Thus, the predictive indicator may be formed from binary, categorical or numeric data.

The third machine-learning method may, for instance, be a multilayer perceptron or other feedforward neural network. However, other suitable machine-learning methods can be used in alternative examples.

In some examples, the workflow 100 further comprises processing the non-image data of the individual using a fourth machine-learning method 160 to produce a third feature vector 165. The fusion algorithm 140 may be configured to fuse the first 125, second 135 and third 165 feature vectors to produce the fused feature vector 145. In this instance, the fused feature vector 145 represents the information of the first feature vector, the second feature vector and the third feature vector.

The fourth machine-learning method is separate and/or different to the first and second machine-learning methods (as well as the third machine-learning method).

In some examples, the second feature vector represents (the) static information of the individual. As previously mentioned, static information is information that does not substantially change over the course of a medical treatment. The third feature vector may represent dynamic information of the individual. As previously mentioned, the dynamic information may comprise or be information that is expected to change or vary throughout the course of a medical treatment.

This approach allows different forms or types of machine-learning algorithm to be used in processing different types of data to characterize or represent different types of data in different feature vector. For instance, a feature vector representing static information may be produced using a fully convolutional network as the second machine-learning method, which facilitates the capture or characterization of links between different static variables of the individual within a feature vector. As another example, a long short-term memory (LSTM) may be used as the fourth machine-learning method to improve the characterization or representation of time dependent (i.e., dynamic) information in a feature vector.

Thus, use of both the second and fourth machine-learning methods can improve the characterization of individual information in feature vectors, and therefore the accuracy of the overall workflow.

It will be appreciated that the first, second and third machine-learning algorithms, together with the fusion algorithm (and fourth machine-learning algorithm if present), effectively represent sub-modules or sub-processes of a larger machine-learning algorithm.

A machine-learning algorithm is any self-training algorithm that processes input data in order to produce or predict output data. Here, for the larger/overall machine-learning algorithm, the input data comprises the individual data of the individual and the output data comprises the predictive indicator (of whether or not the individual has acute respiratory distress syndrome).

Suitable machine-learning algorithms for being employed (e.g., as any of the machine-learning algorithms, including the fusion algorithm) in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives. Embodiments are particularly advantageous when each machine-learning algorithm is a neural network.

The structure of a neural network (sometimes called an artificial neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. $\pm$1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

Training the first, second and third machine-learning algorithms (and optionally the fusion algorithm and/or fourth machine-learning algorithm, if present) can take place using a training dataset in which the training input data entries correspond to example instances of individual data and the training output data entries provide a (ground-truth) predictive indicator for each instance individual data. Each instance of individual data is processed (e.g., according to the previously mentioned workflow), and a predicted predictive indicator is generated for each instance of individual data. Each error between the predicted predictive indicator and the corresponding (ground-truth) predictive indicator is used to modify at least the first, second and third (and, if present, fourth) machine-learning algorithms. The same error may, in some instances, also be used in the training of the fusion algorithm.

This technique follows well-established training procedures for machine-learning algorithms, and other approaches will be appreciated by the skilled person.

The present disclosure provides (e.g., additional) techniques for improving the performance of the fusion algorithm 140, such that the fused feature vector 145 more accurately represents the information of or contained in the first feature vector 125 and/or the second feature vector 135 (and/or the third feature vector 165 if present) and/or of the individual. An improvement to the fusion algorithm will improve the accuracy of the predictive indicator 190, by reducing a risk of error or reducing the introduction of any error in the fusion process.

More particularly, the present disclosure proposes an approach in which the fusion algorithm is trained using at least one measure of loss produced by at least one loss function. Each loss function processes a plurality of training fused feature vectors, independently of the third machine-learning algorithm, to produce a respective measure of loss.

Put another way, the measure of loss produced by any of the herein proposed loss functions (used to train the fusion algorithm) is independent of any predictive indicator produced by the third machine-learning algorithm processing any fused vector produced by the fusion algorithm. Thus, any changes to the third-machine-learning algorithm (e.g., during training) have no effect on any of the measures of loss produced by any of the herein proposed loss function.

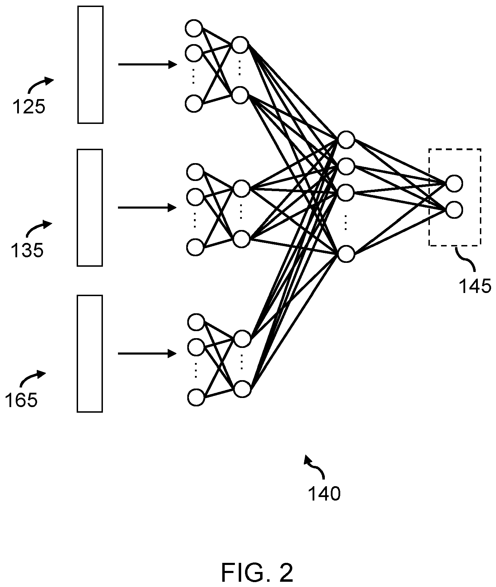

Fig. 2 illustrates an example of a fusion algorithm 140 for improved contextual understanding. The fusion algorithm 140 is configured to fuse a plurality of feature vectors 125, 135, 165 to form a fused feature vector 145. The fused feature vector 145 represents or contains information found in the plurality of feature vectors. In particular, the fused feature vector may be designed (via appropriate training) to contain those features important or required for accurate prediction of ARDS.

In the illustrated example, the plurality of feature vectors includes at least the first 125 and second 135 feature vectors previously described, and optionally the third feature vector 165.

The illustrated fusion algorithm is an autoencoder neural network. Thus, each feature vector 125, 135, 165 is fed as an input to the fusion algorithm. The fusion algorithm then uses a series or sequence of connected layers to produce a (smaller in some embodiments) fused feature vector 145. In particular, each value in the fused feature vector 145 may be at least partially responsive to each value of each feature vector fed as input to the fusion algorithm. The autoencoder neural network is a type of neural network, examples of which have been previously described.

As previously mentioned, the proposed loss function(s) make use of a plurality of training fused feature vectors. This plurality of training fused feature vectors may be produced from a first plurality of instances of training individual data comprising training medical image data and training non-image medical data.

For instance, the plurality of training fused feature vectors may be produced by, for each instance of training individual data, processing the training medical image data using the first machine-learning method to produce a training first feature vector; processing the training non-image medical data using the second machine-learning method to produce a training second feature vector; and fusing the training first feature vector and the training second feature vector using the fusion algorithm to produce the training fused feature vector.

Of course, if a fourth machine-learning method is to be used, then (for each instance of training individual data) the corresponding training fused feature vector may be produced by further processing the training non-image medical data using the fourth-machine learning method to produce a training third feature vector, and adapting the step of fusing to include fusing the first, second and third feature vectors to produce the training fused feature vector.

A loss function processes at least part of the plurality of trained fused feature vectors in order to produce a measure of loss. The measure of loss can be used to update or modify the fusion algorithm, e.g., modify one or more weights or other parameter values of the fusion algorithm.

Each time the fusion algorithm is updated, the plurality of training fused feature vectors may be regenerated using the updated fusion algorithm. The loss function(s) may then redetermine the measure(s) of loss for the plurality of training fused feature vectors. This allows for changes to the fusion algorithm to propagate through to the training fused feature vectors for improved accuracy.

A first loss function for a fusion algorithm produces a first measure of loss that penalizes differences between fused feature vectors of similar individuals (e.g., individuals having a similar or same predictive indicator of ARDS). This first measure of loss can be labelled a first contrastive loss $L_{contrastive1}$.

To produce a first contrastive loss, each training fused feature vector is associated with a respective individual and a respective predictive indicator that indicates whether or not the respective individual has acute respiratory distress syndrome. This association or correspondence may be indicated in a dataset containing the plurality of training fused feature vectors. The predictive indicator in this scenario may be a ground-truth indicator (e.g., defined or determined by an appropriately skilled clinician).

The first loss function determines, for each training fused feature vector in a first subset of the plurality of training fused feature vectors, a first measure of difference between said training fused feature vector and another training fused feature vector associated with a same or similar predictive indicator to the predictive indicator of said fused feature vector.

The precise criterion/criteria for the predictive indicators of two training fused feature vectors to be considered similar may depend upon the nature or data format of the predictive indicator. For instance, two training fused feature vectors may be considered similar if a binary or categorical predictive indicator associated with each training fused feature vector has the same value. As another example, two training fused feature vectors may be considered similar if a difference between the numerical predictive indicator associated with each training fused feature vector is less than a predetermined value or percentage (e.g., they differ by less than 10%).

The first loss function then sums or averages the determined first measures of difference to produce the first measure of loss.

Equation (1) represents an approach for the first loss function, i.e., an approach for defining or determining the first measure of loss $L_{contrastive1}$.

$$L_{contrastive1} = \sum_i max\left(0, ||A_i - A_i{}^+||_2^2\right) \qquad (1)$$

where $A_i$ represents the $i_{th}$ fused feature vector of the first subset. The fused feature vector $A_i$ and positive sample feature vector $A_i{}^+$ belong to the same class (e.g., have similar or the same predictive indicators). The function $||\cdot||$ is any suitable loss or difference function, such as an L1 loss or an L2 loss function.

The fusion algorithm can be trained, using the first measure of loss $L_{contrastive1}$, e.g., to target decreasing or reducing the first measure of loss. Approaches for training an algorithm have been previously described, but typically comprise modifying the value of one or more parameters of the algorithm (e.g., using a gradient descent approach).

A second loss function for a fusion algorithm produces a second measure of loss that penalizes similarities between fused feature vectors of different individuals (e.g., individuals having dissimilar predictive indicators of ARDS). This second measure of loss can be labelled a second contrastive loss $L_{contrastive2}$.

To produce a second contrastive loss, each training fused feature vector remains associated with a respective individual and a respective predictive indicator that indicates whether or not the respective individual has acute respiratory distress syndrome. Once again, the predictive indicator in this scenario may be a ground-truth indicator (e.g., defined or determined by an appropriately skilled clinician).

The second loss function determines, for each training fused feature vector in a second subset of the plurality of training fused feature vectors, a second measure of difference between said training fused feature vector and another training fused feature vector associated with a different or dissimilar predictive indicator to the predictive indicator of said fused feature vector.

The precise criterion/criteria for the predictive indicators of two training fused feature vectors to be considered different or dissimilar may depend upon the nature or data format of the predictive indicator. For instance, two training fused feature vectors may be considered different or dissimilar if the binary or categorical predictive indicators associated with the training fused feature vectors have different values. As another example, two training fused feature vectors may be considered different or dissimilar if a difference between the numerical predictive indicator associated with each training fused feature vector is greater than a predetermined value or percentage (e.g., they differ by less than 10%).

The second loss function will then sum or average the determined second measures of difference to produce a second measure of loss $L_{constractive2}$.

Equation (2) represents an approach for the second loss function, i.e., an approach for defining or determining the second measure of loss $L_{contrastive2}$.

$$L_{contrastive2} = \sum_i max\left(0, ||A_i - A_i^-||_2^2\right) \tag{2}$$

where $A_i$ represents the $i_{th}$ fused feature vector of the second subset. The fused feature vector $A_i$ and positive sample feature vector $A_i^-$ belong to different classes (e.g., have dissimilar predictive indicators). The function $||\cdot||$ is any suitable loss or difference function, such as an L1 loss or an L2 loss function.

The fusion algorithm can be trained using the second measure of loss $L_{contrastive2}$, e.g., to target increasing the second measure of loss. Approaches for training an algorithm have been previously described, but typically comprise modifying the value of one or more parameters of the algorithm (e.g., using a gradient descent approach).

Equation (3) represents a combined loss function, which combines the first and second loss functions previously described to produce a combined measure of loss $L_{combined}$.

$$L_{combined} = \sum_i max\left(0, ||A_i - A_i^+||_2^2 - ||A_i - A_i^-||_2^2 + m\right) \tag{3}$$

The margin parameter m controls distances between similar and dissimilar pairs. The combined loss $L_{combined}$ aims to bring samples from the same class closer and push samples from different class farther away based on these distances.

A third loss function aims to characterize, in the form of a third measure of loss, the capability of a fused feature vector (produced by the fusion algorithm) to reconstruct the first, second and (if present) third feature vector(s). Thus, the third loss function aims to characterize how accurately or appropriately the fused feature vector represents the information contained in the first, second and/or third feature vectors (e.g., or whether information is loss). Use of such a loss function would improve the performance of the fusion algorithm.

The third measure of loss can therefore be labelled a reconstruction loss $L_{reconstruction}$. Use of the reconstruction loss checks or measures whether the fused feature vector will have maintained the salient features of the individual data.

The third measure of loss may be produced by processing each training fused feature vector in a third subset of the plurality of training fused feature vectors (previously described).

More particularly, determining the third measure of loss may comprise processing the training fused feature vector using a reconstruction algorithm to produce a reconstructed first feature vector and a reconstructed second feature vector; determining a third measure of difference between the reconstructed first feature vector and the training first feature vector that was fused to produce the training fused feature vector; and determining a fourth measure of difference between the reconstructed second feature vector and the training second feature vector that was fused to produce the training fused feature vector, and summing and/or averaging each third measure of difference and/or fourth measure of difference to produce a third measure of loss.

If the overall workflow makes use of a fourth machine-learning method, then the reconstruction algorithm may further produce a reconstructed fourth feature vector. Determining the third measure of loss may comprise determining a fifth measure of difference between the reconstructed third feature vector and the training third feature vector that was fused to produce the training fused feature vector. The summing and/or averaging may comprise averaging each third, fourth and/or fifth measure of difference to produce the third measure of loss.

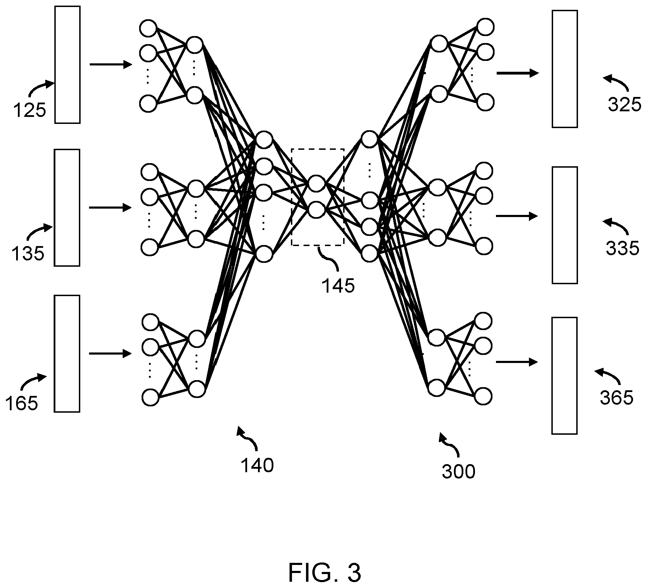

Fig. 3 illustrates an approach for generating the reconstructed feature vectors. In particular, Fig. 3 illustrates the fusion algorithm 140 together with the reconstruction algorithm 300. The reconstruction algorithm processes a fused feature vector 145 to produce the reconstructed feature vectors 325, 335, 365.

The reconstruction algorithm may effectively act as the inverse to the fusion algorithm. Approaches for forming such a reconstruction algorithm will be apparent to the appropriately skilled person. In particular, the reconstruction algorithm may be a trainable algorithm (e.g., trained using the third measure of loss), such as a neural network, that processes the fused feature vector to reconstruct the feature vectors originally used to produce the fused feature vector.

Equation (4) represents a reconstructive loss function that produces the third measure of loss, i.e., the reconstruction loss $L_{reconstruction}$.

$$L_{reconstruction} = \frac{1}{N} \sum_{i=1}^{N} (\| I_i^v, \hat{I}_i^v \| + \| T_i^v, \hat{T}_i^v \| + \| S_i^v, \hat{S}_i^v \|) \tag{4}$$

where:

$I_i^v$ represents the first feature vector 125 used in producing the $i_{th}$ training fused feature vector;

$\hat{I}_i^v$ represents the reconstructed first feature vector 325 (produced using the $i_{th}$ training fused feature vector);

$T_i^v$ represents the second feature vector 135 used in producing the $i_{th}$ training fused feature vector;

$\hat{T}_i^v$ represents the reconstructed second feature vector 335 (produced using the $i_{th}$ training fused feature vector);

$S_i^v$ represents the third feature vector 165 used in producing the $i_{th}$ training fused feature vector;

$\hat{S}_i^v$ represents the reconstructed third feature vector 365 (produced using the $i_{th}$ training fused feature vector);

N is the number of training fused feature vectors in the third subset of the plurality of training fused feature vectors; and

the function $\|\cdot\|$ is any suitable loss or difference function, such as an L1 loss function or an L2 loss function.

In equation (4), the term $\| S_i^v, \hat{S}_i^v \|$ may be omitted in embodiments in which no third feature vector is generated.

The fusion algorithm may be trained to target decreasing the third measure of loss.

The above-described examples of loss functions are independent of the third machine-learning algorithm.

However, the fusion algorithm may be (further) trained using one or more further measures of loss that make use of the third machine-learning algorithm. One example of a further measure of loss is a measure of loss for generating the predictive indicator with a given first and second (and, if used, third) feature vector.

Thus, there is also provided a further loss function is configured to process a plurality of instances of training data, each instance of training data comprising training feature vectors (comprising a training first, second and (if used) third feature vector) and a training predictive indicator that indicates whether or not the training individual associated with the training feature vectors has acute respiratory distress syndrome (which may be ground-truth data).

The further loss function comprises, for each instance of training data: processing the training feature vectors using the fusion algorithm, to produce an intermediate fused feature vector; and processing the intermediate fused feature vector to produce an intermediate predictive indicator. The further loss function also comprises, for each instance of training data, determining a fifth measure of difference between the intermediate predictive indicator and the training predictive indicator. The further loss function also comprises summing and/or average each fifth measure of difference to produce a further measure of loss.

This further measure of loss may be labelled a classification loss $L_{classificaiton}$.

Equation (5) represents an example of such a further loss function, or classification loss function, that produces the further measure of loss, i.e., a classification loss $L_{classification}$.

$$L_{classification} = -\frac{1}{N} \sum_{i=1}^{N} p(y_i | I_i, T_i, S_i) \tag{5}$$

where N represents the number of instances of training data, $I_i^v$ represents the first feature vector of the ith instance of training data; $T_i^v$ represents the second feature vector of the ith instance of training data; Si represents the third feature vector of the ith instance of training data (which may be omitted); $y_i$ represents the predictive indicator of the ith instance of training data. The function p(·) is any suitable probability or error function.

Some example approaches for training of a fusion algorithm make use of all the above-described measures of loss, e.g., to create an overall measure of loss for the fusion algorithm. Put another way, some methods for training a fusion algorithm combine the above-described measures of loss (e.g., perform a sum, weighted sum, average or weighted average) of the described measures of loss.

This overall measure of loss $L_{overall}$ thereby consists of a classification loss, a contrastive loss, and a reconstruction loss, and provides a technique for leveraging both the inter-class similarity and the cross-modality information for addressing multi-modal problems.

Equation (6) represents an example of an approach for producing an overall measure of loss $L_{overall}$:

$$L_{overall} = L_{classification} + \alpha.L_{combined} + \beta.L_{reconstruction} \qquad (5)$$

factors $\alpha$ and $\beta$ are employed to regulate the impact of the inter-modality task and reconstruction task, respectively. The value of the factors $\alpha$ and $\beta$ may be controlled, for example, based on the particular use-case scenario and/or a user input.

Experimentation has been performed on the effectiveness of using the proposed workflow (in which at least the fusion model is trained using the overall measure of loss). The publicly available MIMIC-CXR and MIMIC-IV datasets were used to train and test three ARDS detection models. Both databases used the same patient IDs, stays, admissions and the dates and times are consistent for each patient. The three ARDS detection models include medical image data (MID) classification with DenseNet121, non-image medical data (NIMD) classification with XGBoost, and using the proposed workflow to generate the predictive indicator.

Table 1 illustrates the effectiveness (AUROC) of each model, together with the data types used by each model to produce the predictive indicator.

TABLE 1

| Model | Data Type | AUROC |
|---|---|---|
| XGBoost | NIMD | 0.68 |
| DenseNet121 | MID | 0.84 |
| Proposed Approach | MID + NIMD | 0.88 |

The first machine-learning algorithm, the second machine-learning algorithm and/or the third machine-learning algorithm (and/or, if present, the fourth machine-learning algorithm) may also be trained using the at least one measure of loss produced by any herein proposed loss function. Thus, any herein proposed loss function can be integrated into a training procedure for the third, second, third and/or (if present) fourth machine-learning algorithms.

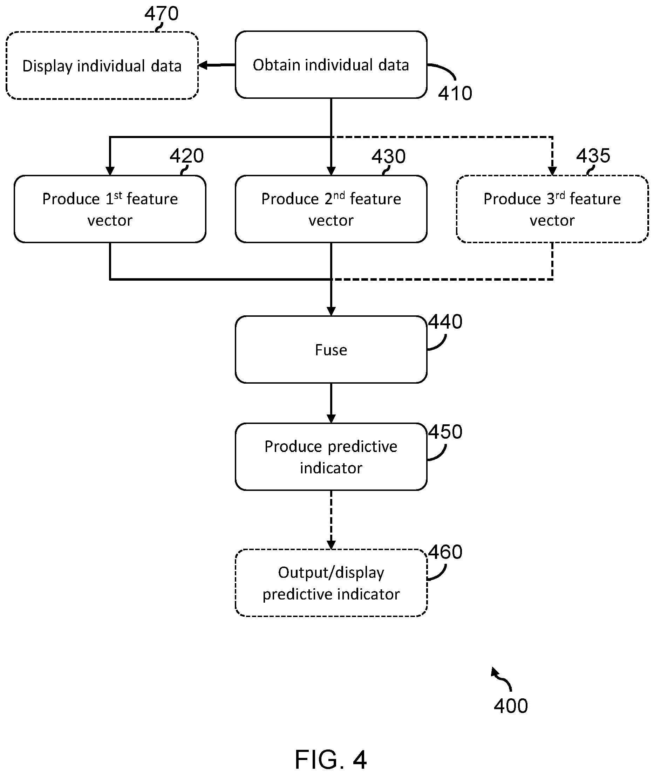

Fig. 4 is a flowchart illustrating a computer-implemented method 400 that employs the herein proposed approach. Thus, the computer-implemented method is configured for generating a predictive indicator of acute respiratory distress syndrome for an individual.

The computer-implemented method 400 comprises a step 410 of obtaining individual data of the individual. As previously explained, the individual data comprises at least medical image data of the individual; and non-image medical data of the individual.

The computer-implemented method 400 also comprises a step 420 of processing the medical image data using a first machine-learning method to produce a first feature vector.

The computer-implemented method also comprises a step 430 of processing the non-image data of the individual using a second machine-learning method to produce a second feature vector.

Optionally, the computer-implemented method comprises a step 435 of processing the non-image data using a fourth machine-learning method to produce a third feature vector. In such embodiments, the second feature vector may represent static information of the individual, being information that does not substantially change over the course of a medical treatment; and the third feature vector may represent dynamic information of the individual, being information that is expected to change or vary throughout the course of a medical treatment.

The computer-implemented method 400 also comprises a step 440 of fusing the first feature vector and the second feature vector, using a fusion algorithm. The fusion algorithm forms a fused feature vector representing the information of both the first feature vector and the second feature vector.

Where step 435 is performed, step 440 may comprise fusing the first feature vector, the second feature vector and the third feature vector, using the fusion algorithm, to form the fused feature vector to represent the information of the first feature vector, the second feature vector and the third feature vector.

The computer-implemented method also comprises a step 450 of processing the fused feature vector using a third machine-learning algorithm to generate a predictive indicator that indicates whether or not the individual has acute respiratory distress syndrome.

As previously explained, the fusion algorithm is a trainable algorithm and is trained using at least one measure of loss produced by at least one loss function. Each loss function processes a plurality of training fused feature vectors, independently of the third machine-learning algorithm, to produce a respective measure of loss.

The method 400 may further comprise a step 460 of outputting the predictive indicator. Step 460 may, for instance, comprise outputting the predictive indicator to a further processing system or a memory.

In some examples, step 460 comprises controlling a user interface to provide a user-perceptible output of the predictive indicator. This provides a user of the user interface with an indicator of likely ARDS, aiding them to make a clinical decision about the individual (whose individual data was obtained in step 410).

Steps 410 to 450 may be iteratively repeated, e.g., to iteratively update based on any changes to the individual data over time. In such embodiments, step 460 may comprise controlling a user interface to provide a user-perceptible output of the predictive indicator together with historic indicators (e.g., predictive indicators generated in previously iterations of steps 410 to 450). This can advantageously allow a user (e.g., a clinician) to view any changes in the predicted likelihood of ARDS to identify potential deterioration of the individual's condition, e.g., to trigger the performance of an intervention.

Other uses for a predictive indicator of ARDS will be apparent to the appropriately skilled person, and are not disclosed in detail for the sake of conciseness. For instance, the predictive indicator of ARDS may be provided as an input for a further processing method to perform a further automated clinical analysis process.

The method 400 may further comprise a step 470 of controlling a user interface to provide a user-perceptible output of a portion of the individual data. This provides a user of the user interface with information useful for confirming or checking the accuracy of the indicator of likely ARDS. This helps the user in making a clinical decision about the individual (whose individual data was obtained in step 410).

There is also proposed a computer-implemented method for training a fusion algorithm for use in the method 400. The computer-implemented method comprises training the fusion algorithm using at least one measure of loss produced by at least one loss function that processes a plurality of training fused feature vectors independently of the third machine-learning algorithm. Detailed approaches for training the fusion algorithm and examples of suitable measures of loss have been previously described.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

There is also proposed a user interface system that comprises any such processing system and a user interface communicatively coupled to the processing system. The processing system may be configured to control an operation of the user interface to provide one or more user-perceptible outputs, e.g., of the predictive indicator and/or historic predictive indicators.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. A computer-implemented method of generating a predictive indicator of acute respiratory distress syndrome for an individual, the computer-implemented method comprising:

   obtaining individual data of the individual comprising:

      medical image data of the individual; and
      non-image medical data of the individual;

   processing the medical image data using a first machine-learning method to produce a first feature vector;
   processing the non-image data of the individual using a second machine-learning method to produce a second feature vector;
   fusing the first feature vector and the second feature vector, using a fusion algorithm, to form a fused feature vector representing the information of both the first feature vector and the second feature vector; and
   processing the fused feature vector using a third machine-learning algorithm to generate a predictive indicator that indicates whether or not the individual has acute respiratory distress syndrome,
   wherein the fusion algorithm is a trainable algorithm and is trained using at least one measure of loss produced by at least one loss function,
   wherein each loss function processes a plurality of training fused feature vectors, independently of the third machine-learning algorithm, to produce a respective measure of loss.

2. The computer-implemented method of claim 1, wherein the fusion algorithm comprises an autoencoder neural network.

3. The computer-implemented method of claim 1 or 2, wherein each training fused feature vector is associated with a respective individual and a respective predictive indicator that indicates whether or not the respective individual has acute respiratory distress syndrome.

4. The computer-implemented method of claim 3, wherein the at least one loss function comprises a first loss function that:

   determines, for each training fused feature vector in a first subset of the plurality of training fused feature vectors, a first measure of difference between said training fused feature vector and another training fused feature vector associated with a same or similar predictive indicator to the predictive indicator of said fused feature vector; and
   sums or averages the determined first measures of difference to produce a first measure of loss.

5. The computer-implemented method of claim 4, wherein the fusion algorithm is trained to target decreasing the first measure of loss.

**6.** The computer-implemented method of any of claims 3 to 5, wherein the at least one loss function comprises a second loss function that:

> determines, for each training fused feature vector in a second subset of the plurality of training fused feature vectors, a second measure of difference between said training fused feature vector and another training fused feature vector associated with a different predictive indicator to the predictive indicator of said fused feature vector; and
>
> sums or averages the determined second measures of difference to produce a second measure of loss.

**7.** The computer-implemented method of claim 6, wherein the fusion algorithm is trained to target increasing the second measure of loss.

**8.** The computer-implemented method of any of claims 1 to 7, wherein the plurality of training fused feature vectors is produced by, for each of a first plurality of instances of training individual data comprising training medical image data and training non-image medical data:

> processing the training medical image data using the first machine-learning method to produce a training first feature vector;
>
> processing the training non-image medical data using the second machine-learning method to produce a training second feature vector; and
>
> fusing the training first feature vector and the training second feature vector using the fusion algorithm to produce the training fused feature vector.

**9.** The computer-implemented method of claim 8, wherein the at least one loss function comprises a third loss function that:

> for each training fused feature vector in a third subset of the plurality of training fused feature vectors:
>
> > processing the training fused feature vector using a reconstruction algorithm to produce a reconstructed first feature vector and a reconstructed second feature vector;
> >
> > determining a third measure of difference between the reconstructed first feature vector and the training first feature vector that was fused to produce the training fused feature vector;
> >
> > determining a fourth measure of difference between the reconstructed second feature vector and the training second feature vector that was fused to produce the training fused feature vector; and
>
> summing and/or averaging each third measure of difference and/or fourth measure of difference to produce a third measure of loss.

**10.** The computer-implemented method of any of claims 1 to 9, wherein:

> the fusion algorithm is trained using at least one further measure of loss produced by a further loss function;
> the further loss function is configured to process a plurality of instances of training data, each instance of training data comprising:
>
> > training individual data comprising training medical image data and training non-image medical data of a training subject; and
> > a training predictive indicator that indicates whether or not the training individual has acute respiratory distress syndrome,
>
> the further loss function comprises:
>
> > for each instance of training data:
> >
> > > processing the training medical image data and the training non-image medical data, using the first machine-learning method, the second machine-learning method, the fusion algorithm and the third machine-learning method, to produce an intermediate predictive indicator;
> > > determining a fifth measure of difference between the intermediate predictive indicator and the training predictive indicator; and

summing and/or average each fifth measure of difference to produce a further measure of loss.

11. The computer-implemented method of any of claims 1 to 10, further comprising processing the non-image data of the individual using a fourth machine-learning method to produce a third feature vector, wherein

fusing the first feature vector and the second feature vector comprises fusing the first feature vector, the second feature vector and the third feature vector, using a fusion algorithm, to form a fused feature vector to represent the information of the first feature vector, the second feature vector and the third feature vector;
the second feature vector represents static information of the individual, being information that does not substantially change over the course of a medical treatment; and
the third feature vector represents dynamic information of the individual, being information that is expected to change throughout the course of a medical treatment.

12. The computer-implemented method of any of claims 1 to 11, wherein the first machine-learning algorithm, the second machine-learning algorithm and/or the third machine-learning algorithm is trained using the at least one measure of loss produced by the at least one loss function.

13. A computer-implemented method for training a fusion algorithm for use in the method of any of claims 1 to 12, the computer-implemented method comprising training the fusion algorithm using at least one measure of loss produced by at least one loss function that processes a plurality of training fused feature vectors independently of the third machine-learning algorithm.

14. A computer program product comprising computer program code means which, when executed on a computing device having a processing system, cause the processing system to perform all of the steps of the method according to any of claims 1 to 13.

15. A processing system for generating a predictive indicator of acute respiratory distress syndrome for an individual, the processing system being configured to:

obtain individual data of the individual comprising:

medical image data of the individual; and
non-image medical data of the individual;

process the medical image data using a first machine-learning method to produce a first feature vector;
process the non-image data of the individual using a second machine-learning method to produce a second feature vector;
fuse the first feature vector and the second feature vector, using a fusion algorithm, to form a fused feature vector to represent the information of the first feature vector and the second feature vector; and
process the fused feature vector using a third machine-learning algorithm to generate a predictive indicator that indicates whether or not the individual has acute respiratory distress syndrome,
wherein the fusion algorithm is a trainable algorithm and is trained using at least one measure of loss produced by at least one loss function,
wherein each loss function processes a plurality of training fused feature vectors, independently of the third machine-learning algorithm, to produce a respective measure of loss.

FIG. 1

FIG. 2

FIG. 3

470

Display individual data ← Obtain individual data

410

420
Produce 1st feature vector

430
Produce 2nd feature vector

435
Produce 3rd feature vector

440
Fuse

450
Produce predictive indicator

460
Output/display predictive indicator

400

FIG. 4

Europäisches
Patentamt

European
Patent Office

Office européen
des brevets

# EUROPEAN SEARCH REPORT

Application Number

EP 23 19 5897

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CABAN JESUS J ET AL: "Enhancing image classification models with multi-modal biomarkers", MEDICAL IMAGING 2011: COMPUTER-AIDED DIAGNOSIS, SPIE, 1000 20TH ST. BELLINGHAM WA 98225-6705 USA, vol. 7963, no. 1, 3 March 2011 (2011-03-03), pages 1-9, XP060008454, DOI: 10.1117/12.878084 [retrieved on 2011-03-08] * page 2, paragraphs 5, 9 * * page 3, paragraph 2 * | 1-15 | INV. G16H15/00 G16H30/40 G16H50/20 |
| A | ILHAN HAMZA OSMAN ET AL: "Decision and feature level fusion of deep features extracted from public COVID-19 data-sets", APPLIED INTELLIGENCE, KLUWER ACADEMIC PUBLISHERS, DORDRECHT, NL, vol. 52, no. 8, 30 October 2021 (2021-10-30), pages 8551-8571, XP037857655, ISSN: 0924-669X, DOI: 10.1007/S10489-021-02945-8 [retrieved on 2021-10-30] * the whole document * | 1-15 | |
| A | AL MOHIDUR RAHMAN PORAG ET AL: "A Comparison Study of Deep CNN Architecture in Detecting of Pneumonia", ARXIV.ORG, CORNELL UNIVERSITY LIBRARY, 201 OLIN LIBRARY CORNELL UNIVERSITY ITHACA, NY 14853, 30 December 2022 (2022-12-30), XP091405098, * page 3, paragraph 5 * | 1-15 | |

TECHNICAL FIELDS
SEARCHED      (IPC)

G16H

The present search report has been drawn up for all claims

| Place of search | Date of completion of the search | Examiner |
|---|---|---|
| Munich | 28 February 2024 | Laub, Christoph |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)